# EUROPEAN PATENT APPLICATION

(11) **EP 4 374 788 A1**
(43) Date of publication of application: **29.05.2024**
(21) Application number: 21951046.8
(22) Date of filing: 23.11.2021
(51) Int. Cl.: A61B 5/145, A61B 5/00, A61B 5/1459, A61B 5/1455, A61B 5/1495, A61B 5/07

(54) **BIOMETRIC INFORMATION MEASUREMENT DEVICE AND METHOD IN CONSIDERATION OF SENSOR STATE MEASUREMENT**

(30) Priority: 19.07.2021 KR 20210094404
(71) Applicant: SB Solutions Inc., Ulsan 44919 (KR)
(72) Inventor: SEO, Seungup, Ulju-gun Ulsan 44919 (KR); SUNG, Namhwan, Ulju-gun Ulsan 44919 (KR)
(74) Representative: AWA Sweden AB
(86) International application number: PCT/KR2021/017214
(87) International publication number: WO 2023/003098

(57) **Abstract**

Disclosed is a device and method for measuring biometric information considering sensor condition measurement. A biometric information measurement device according to an example embodiment may include an implant device configured to insert into a body of a subject to be analyzed, to emit an electromagnetic wave of a specific frequency, and to measure a signal reflected from a surrounding analyte; and a non-invasive measurement device configured to measure a change in a field formed by the electromagnetic wave of the specific frequency outside the body of the subject to be analyzed.

## Description

### TECHNICAL FIELD

The following description relates to a device and method for measuring biometric information considering sensor condition measurement.

### RELATED ART

The number of adult diseases, such as diabetes, hyperlipidemia, and thrombosis, continuously increases. Since it is important to continuously monitor and manage the diseases, the diseases need to be periodically measured using various biosensors. A typical type of biosensor involves injecting blood taken from a finger into a test strip and then quantifying an output signal using an electrochemical method or a photometric method. This approach requires blood collection each time, which causes a lot of pain to a user.

For example, measuring blood sugar is most basic to manage diabetes of hundreds of millions of people around the world. Therefore, a blood sugar measurement device is an essential diagnostic device for diabetic patients. Recently, various blood sugar measurement devices are developed, but a method of collecting blood by pricking a finger and directly measuring the concentration of glucose in the blood is most commonly used. When using an invasive method, there is a method of measuring blood sugar by infiltrating an invasive sensor into the skin, measuring the blood sugar for a certain period of time, and then recognizing the same with an external reader.

On the other hand, a non-invasive method includes a method using light-emitting diode (LED)-photo diode (PD). However, since the non-invasive method is attached to the skin, its accuracy decreases due to an environmental factor, such as sweat and temperature, and foreign substances.

The aforementioned information is provided only for understanding and may include contents that do not form a part of the related art and may not include what the related art may present to one of ordinary skill in the art.

### DETAILED DESCRIPTION

### TECHNICAL SUBJECT

Example embodiments provide a device and method for measuring biometric information that may include an implant device configured to insert into a body of a subject to be analyzed and to measure biometric information.

### TECHNICAL SOLUTION

Provided is a sensor information measurement device including an implant device configured to insert into a body of a subject to be analyzed to emit an electromagnetic wave of a specific frequency, and to measure a signal reflected from a surrounding analyte; and a non-invasive measurement device configured to measure a change in a field formed by the electromagnetic wave of the specific frequency outside the body of the subject to be analyzed.

According to an aspect, the non-invasive measurement device may be configured to measure a change in intensity of the field that changes according to a change in permittivity in the body of the subject to be analyzed.

According to another aspect, the non-invasive measurement device may be configured to process at least one of correction on measurement data of the implant device and measurement of an operating state of the implant device based on the measured change in the field.

According to still another aspect, the non-invasive measurement device may be configured to determine an abnormality status of the implant device according to the operating state of the implant device and to provide an alarm to a user through the sensor information measurement device or a terminal that communicates with the sensor information measurement device depending on the determined abnormality status.

According to still another aspect, the sensor information measurement device may further include an external device configured to supply power to the implant device, to receive first measurement data of the implant device, to receive the change in the field measured by the non-invasive measurement device, and to process at least one of correction on measurement data of the implant device and measurement of an operating state of the implant device based on the received change in the field.

According to still another aspect, the external device may include a communication unit configured to communicate with the implant device; and a position adjustment tool configured to adjust a position of the communication unit in the external device based on position state information with the implant device.

According to still another aspect, the position state information may include information on a degree of alignment between the external device and the implant device, and information on the degree of alignment may be generated based on position detection results of a first position detection sensor included in the implant device and position detection results of a second position detection sensor included in the external device.

According to still another aspect, the external device may be configured to generate an alarm message for requesting a user to relocate the external device if a position state value between a first position of the external device and a second position of the implant device according to the position detection results of the first position detection sensor and the position detection results of the second position detection sensor included in the external device is greater than or equal to a threshold.

According to still another aspect, the implant device may include a fixing tool configured to fix the implant device at an insertion position in the body of the subject to be analyzed.

According to still another aspect, the implant device may include a tool configured to measure a tilt of the implant device based on the skin surface of the subject to be analyzed; and a tool configured to provide an alarm through an external device or a terminal when the measured tilt is tilted by a predetermined tilt or more.

### EFFECT

According to some example embodiments, it is possible to provide a device and method for measuring biometric information that may include an implant device configured to insert into a body of a subject to be analyzed and to measure biometric information.

### BRIEF DESCRIPTION OF DRAWINGS

FIG. 1 is a block diagram illustrating an example of a biometric information measurement device according to an example embodiment of the present invention.
FIG. 2 illustrates an example of three modes of a biometric information measurement device according to an example embodiment of the present invention.
FIG. 3 is a block diagram illustrating another example of a biometric information measurement device according to an example embodiment of the present invention.
FIG. 4 illustrates a front view and a perspective view of a package of an implant device according to an example embodiment of the present invention.
FIGS. 5 and 6 illustrate examples of an arrangement structure of a radio frequency (RF) antenna for measurement and a power receiving coil of an implant device.
FIG. 7 is a flowchart illustrating an example of a biometric information measurement method of an implant device according to an example embodiment of the present invention.
FIG. 8 is a flowchart illustrating an example of a biometric information measurement method of another external device according to an example embodiment of the present invention.

### BEST MODE

Hereinafter, example embodiments are described in detail with reference to the accompanying drawings. However, various modifications may be made to the example embodiments and the scope of the present application is not limited to or restricted by the example embodiments. It should be understood that the example embodiments include all changes, equivalents, and substitutions within the claims.

The terms used herein are used to simply explain specific example embodiments and are not construed to limit the present invention. The singular forms "a," "an," and "the," are intended to include the plural forms as well, unless the context clearly indicates otherwise. It will be further understood that the terms "comprises/comprising (incudes/including)," and "has/having" when used in this specification, specify the presence of stated features, integers, steps, operations, elements, components, or groups thereof but do not preclude the presence or addition of one or more other features, integers, steps, operations, elements, components, and/or groups thereof.

Unless otherwise defined, all terms including technical and scientific terms used herein have the same meaning as commonly understood by one of ordinary skill in the art to which example embodiments belong. Terms, such as those defined in commonly used dictionaries, should be interpreted as having a meaning that is consistent with their meaning in the context of the relevant art and/or this disclosure, and should not be interpreted in an idealized or overly formal sense unless expressly so defined herein.

Also, when describing with reference to the accompanying drawings, like reference numerals refer to like elements throughout the present specification and repeated description related thereto is omitted. When it is determined that detailed description related to the related known art may make the example embodiments unnecessarily ambiguous, the detailed description is omitted.

Also, the terms "first," "second," "A," "B," "(a)," " (b)," etc., may be used herein to describe various components of example embodiments. However, these terms are only used to distinguish one component from another component and essence or turn or order of a corresponding component is not construed by the term. For example, when a component is referred to as being "connected to," "coupled to," or "accessed to" another component, it should be understood that the component may be directly connected to or accessed to the other component, and still another component may be "connected," "coupled," or "accessed" between the components.

A component including a common function with a component included in one example embodiment is described using the same name in another example embodiment. Unless otherwise defined, description related to one example embodiment may also be applied to another example embodiment and detailed description within the overlapping range is omitted.

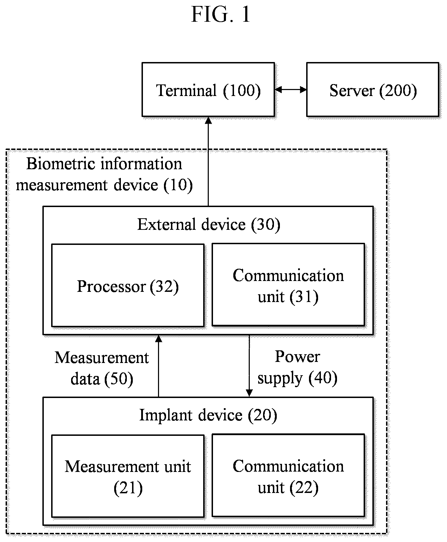

FIG. 1 is a block diagram illustrating an example of a biometric information measurement device according to an example embodiment of the present invention. A biometric information measurement device 10 according to the example embodiment may include an implant device 20 inserted into a body of a subject to be analyzed to measure of which biometric information (e.g., analyte concentration, such as blood sugar, oxygen saturation, etc.) and an external device 30 provided outside the body of the subject to be analyzed at a position corresponding to a position of the implant device 20. The subject to be analyzed may be a human or an animal.

The external device 30 refers to a sensor attached or worn outside the body of the subject to be analyzed and may be fastened to the outside of the body of the subject to be analyzed using various methods, such as a banding method and an adhesive method. The external device 30 may include a communication unit 31, and may provide biometric information to a paired or preset terminal 100 through the communication unit 31.

Depending on example embodiments, the external device 30 may provide the biometric information itself to the terminal 100, and may also variously analyze the biometric information and may provide analysis results, alert, and the like, to the terminal 100. When the biometric information itself is provided to the terminal 100, the terminal 100 may perform various types of analysis on the biometric information. An analysis method of the biometric information may be easily selected by an operator.

Also, the external device 30 may secure measurement accuracy and measurement continuity by blocking a performance change by an external environment. Meanwhile, the external device 30 may improve accuracy by securing complementary data with the implant device 20.

The implant device 20 may be inserted into the body of the subject to be analyzed. For example, the implant device 20 does not directly contact blood or is not provided inside the blood vessel and, instead, may be provided in an area other than the blood vessel at a predetermined depth from the skin of the subject. That is, the implant device 20 may be provided in the subcutaneous area between the skin and the blood vessel.

The implant device 20 may measure the analyte concentration by emitting an electromagnetic wave of a specific frequency and by measuring a signal reflected from an analyte around the sensor. For example, in the case of measuring blood sugar, the implant device 20 may measure the blood sugar by emitting an electromagnetic wave of a specific frequency and by measuring a signal reflected from glucose around the sensor.

The external device 30 may be provided outside the body of the subject to be analyzed at a position corresponding to a position at which the implant device 20 is provided, may perform power supply 40 to the implant device 20, and may receive measurement data 50 (e.g., the aforementioned biometric information) measured by the implant device 20.

If the concentration (e.g., blood sugar level) of analyte in the blood vessel of the subject to be analyzed changes, the concentration of the analyte in the subcutaneous area may change. In this case, permittivity in the subcutaneous area may vary depending on a change in the concentration of the analyte. Here, a resonant frequency in a measurement unit 21 included in the implant device 20 may vary depending on a change in the permittivity in the surrounding subcutaneous area. For example, the measurement unit 21 may include a specific pattern of a conducting wire and a feeder. Here, if the permittivity in the surrounding subcutaneous area varies, capacitance of the measurement unit 21 may also vary and thus, the resonant frequency may also vary due to the specific pattern and the feeder. Since the subcutaneous analyte concentration changes in proportion to the analyte concentration of adjacent blood vessel, the biometric information measurement device 10 may ultimately calculate biometric information, such as analyte concentration, using a resonant frequency corresponding to a change in the subcutaneous permittivity.

In an example embodiment, the biometric information measurement device 10 may also calculate corresponding relative permittivity using a frequency (e.g., resonant frequency) of a point at which magnitude of scattering parameter is smallest or largest.

In an example embodiment, the measurement unit 21 of the implant device 20 may be configured in a form of a resonant element, and the implant device 20 may generate a signal by sweeping a frequency within a predetermined frequency band and may inject the generated signal into the resonant element. Here, the external device 30 may measure the scattering parameter for the resonant element to which a signal with a changing resonant frequency is supplied.

A communication unit 22 of the implant device 20 may transmit data measured by the measurement unit 21 to the external device 30, and the communication unit 21 may receive power for generating a signal supplied to the measurement unit 21 from the external device 30 using a wireless power transmission method.

The external device 30 may include a processor 32 and the communication unit 31, and the communication unit 31 may receive measurement data (e.g., scattering parameter, change level of resonant frequency, etc.) measured by the implant device 20. Here, the processor 32 of the external device 30 may determine the analyte concentration using the measurement data received from the implant device 20. Depending on example embodiments, the analyte concentration may be directly determined by the external device 30 and may be determined by the terminal 100 to which the measurement data is delivered from the external device 30.

In an example embodiment, a look-up table (LUT) in which measurement data (scattering parameter and/or change level of resonant frequency) and the analyte concentration are mapped in advance may be stored in the external device 30. The processor 32 may load the analyte concentration based on the look-up table.

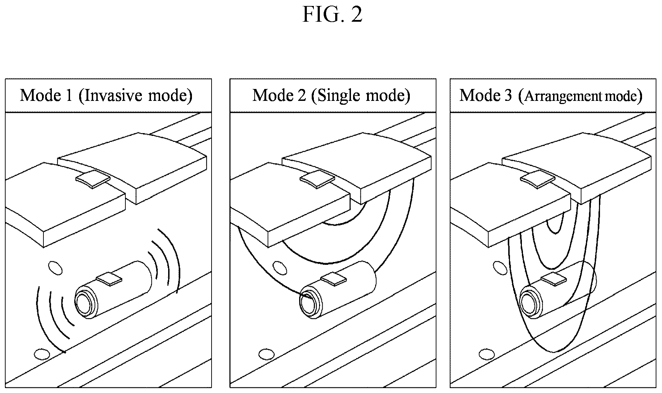

FIG. 2 illustrates an example of three modes of a biometric information measurement device according to an example embodiment of the present invention. The biometric information measurement device 10 according to the example embodiment may operate in three modes. The three modes may be performed independently and may be performed alternately at regular time intervals.

### <Mode 1: invasive mode>

In mode 1, the biometric information measurement device 10 may perform direct measurement on the analyte that diffused from the blood vessel of the subject to the interstitial fluid within the tissue. For example, an IC chip as the measurement unit 21 of the implant device 20 may output an electromagnetic wave with a specific frequency, may emit the analyte, such as glucose around the implant device 20, and may measure a signal reflected back from the analyte. The implant device 20 may output a waveform (e.g., sine wave) of a resonant frequency that changes over time, and, when a reflected signal according to a frequency at a specific time is detected, may generate measurement data for biometric information corresponding to the frequency.

### <Mode 2: single mode>

In mode 2, at least one external device 30 is provided. Desirably, two external device 30 may be configured. Here, in mode 2, the external devices 30 may include a first external device and a second external device arranged at a predetermined interval.

In the biometric information measurement device 10, the first external device and the second external device provided at the predetermined interval may be coupled to measure an electromagnetic wave according to a change in the analyte concentration in the interstitial fluid on the outer surface of the skin of the subject to be analyzed and correction may be performed on a measurement value using measurement data related to the biometric information of the implant device 20. The biometric information measurement device 10 may improve accuracy of measurement of the analyte concentration by performing the correction on the measurement value through multiple modes.

### <Mode 3: arrangement mode>

In the foregoing mode 1, the implant device 20 of the biometric information measurement device 10 emits an electromagnetic wave to the analyte around the implant device 20 and measures a signal that is reflected back from the analyte.

In mode 3, the biometric information measurement device 10 emits an electromagnetic wave that reaches a depth of the blood vessel of the subject to be analyzed and generates measurement data related to biometric information (e.g., blood sugar level as analyte concentration) from the signal reflected back from the analyte in the blood vessel.

In general, when the analyte concentration in the blood vessel changes, the analyte concentration in the subcutaneous area may change and the permittivity in the subcutaneous area varies according to the change in the analyte concentration

In the aforementioned mode 1 and mode 2, measurement data for biometric information is generated by performing measurement on the subcutaneous area. Due to this aspect, there may be some differences between the actual analyte concentration in the blood vessel and the analyte concentration in the subcutaneous area.

Therefore, the biometric information measurement device 10 may acquire measurement data related to biometric information in the actual blood vessel by performing the operation as in mode 3, which may solve a time delay issue of the analyte concentration. Also, since a sudden amount of change in the analyte concentration of the subject to be analyzed through this mode 3, problems that may occur to the subject to be analyzed during the aforementioned time delay may be verified in advance.

Also, the biometric information measurement device 10 according to an example embodiment may perform more accurate biometric information measurement by calibrating a value of the analyte concentration through simultaneous operation of at least two modes among three modes. For example, simultaneously using modes 1 and 2 of the implant device 20, the biometric information measurement device 10 according to the example embodiment may secure accuracy by securing data diversity and may improve accuracy of biometric information measurement through repeated testing.

Also, the biometric information measurement device 10 according to an example embodiment may solve a time delay issue that is a problem of conventional interstitial fluid-based biometric information measurement sensors by monitoring in real time a change in the analyte concentration in the blood vessel through improvement of penetration depth of the electromagnetic wave emitted using mode 3.

Also, using a plurality of sensors and a plurality of modes together as above, it is possible to improve accuracy of a biometric information prediction method and to solve a reproducibility issue by securing data diversity and by adjusting a calibration cycle.

In an example embodiment, the biometric information measurement device 10 may also predict the analyte concentration by linking the measurement data of modes 1, 2, and 3 and measurement data of other sensors (e.g., an environmental sensor, a temperature sensor, a humidity sensor, etc.) with a Bayesian filter-based algorithm.

Also, the biometric information measurement device 10 according to an example embodiment may simultaneously use modes 1 and 2 to secure reproducibility of permittivity measurement, and when analyte concentration measured using measurement data of mode 1 is not the same as analyte concentration measured using measurement data of mode 2, may perform correction by performing re-measurement or by measuring and entering the analyte concentration through blood collection.

As described above, when values of analyte concentration measured in the respective modes are the same by a plurality of pieces of measurement data acquired through multimode measurement, the biometric information measurement device 10 may perform cross-verification on results. Only when values of analyte concentration measured in multiple modes are different, the biometric information measurement device 10 may request the subject to be analyzed to measure the analyte concentration using blood collection. In this manner, the number of blood collections of the subject to be analyzed may be reduced.

### (1) Solving a problem with calibration depending on an individual's condition and disease

In the case of the conventional implantable biometric information measurement method, there is a problem in that biometric information is unilaterally measured without considering an individual's characteristic. For example, conventionally, when measuring biometric information such as analyte concentration, calibration needs to be performed by performing blood collection since various types of conditions and situations differ depending on the individual's condition and disease.

The biometric information measurement device 10 according to example embodiments of the present invention may solve the aforementioned problem through the following example embodiments.

The biometric information measurement device 10 according to an example embodiment may receive a value of first analyte concentration and a value of second analyte concentration of the subject to be analyzed. In detail, when a calibration module of the biometric information measurement device 10 receives the value of first analyte concentration measured in a fasting state of the subject to be analyzed and the value of second analyte concentration measured after a predetermined period of time after the subject to be analyzed consumes (inhales) a preset reference (e.g., candy, pills, etc.) after measuring the value of first analyte concentration, the calibration module may perform calibration for the subject based on the value of first analyte concentration and the value of second analyte concentration.

The biometric information measurement device 10 may include a separate calibration module to perform the above calibration, and may receive the value of first analyte concentration and the value of second analyte concentration and perform calibration. In another example embodiment, the calibration may be performed through a service application installed in the terminal 100 that is paired (connected) to the biometric information measurement device 10.

For example, in the case of measuring blood sugar, the reference the subject consumes in the fasting state may have a certain amount of sugar and there may be an expected blood sugar value expected from a fasting blood sugar value when the subject to be analyzed consumes the reference. In this case, the calibration module may perform calibration on the subject to be analyzed by receiving the value of first analyte concentration (fasting blood sugar value) and the value of second analyte concentration (blood sugar value measured after a predetermined period of time after consuming reference) and by comparing a difference value between the value of first analyte concentration and the value of second analyte concentration to the expected blood sugar value.

For example, the calibration module may calculate characteristics of the subject to be analyzed from the value of first analyte concentration and the value of second analyte concentration along with a disease and a condition of the subject to be analyzed and may adjust a carbohydrate ratio value and/or insulin sensitivity value based on the calculated characteristics.

Also, the calibration module may provide calibration performance data to a server 200 and the server 200 may build bigdata and learning data on how the calibration performance data differs for each piece of information, such as disease and condition of the subject, and may apply the same to a prediction model later for actual use.

In addition, since the implant device 20 is inserted into the body of the subject to be analyzed for its use, various issues may occur over time and the calibration module may perform calibration to prevent measurement errors caused by such issues.

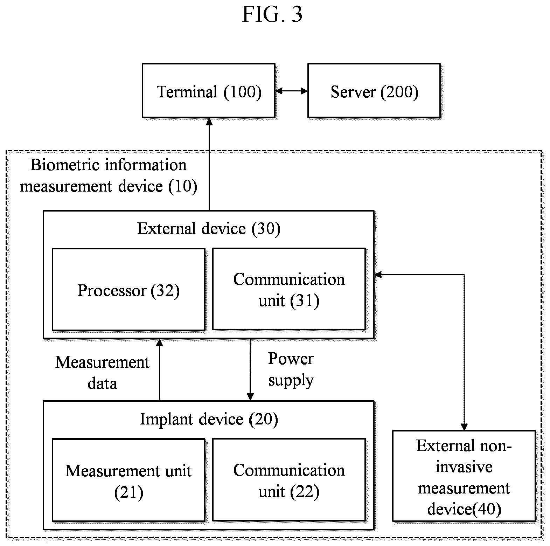

FIG. 3 is a block diagram illustrating another example of a biometric information measurement device according to an example embodiment of the present invention. In another example embodiment, the biometric information measurement device 10 may additionally include an external non-invasive measurement device 40 to check and monitor a state of the implant device 20.

The external non-invasive measurement device 40 may check an operating state of the implant device 20 or may perform calibration based on measured data. For example, the biometric information measurement device 10 may include a charge coupled device (CCD) camera installed outside the skin of the subject as the external non-invasive measurement device 40. In this case, the CCD camera may measure a change in a field that varies in response to a change in permittivity inside the skin and may measure calibration or a sensor operating state using the measured data. Here, the change in the field may represent a change in intensity of the field formed by an electromagnetic wave of a specific frequency emitted from the implant device 20. The calibration may represent correction on measurement data of the implant device 20, and measurement of the sensor operating state may represent measurement for an operating state of the implant device 20. Meanwhile, the correction or the measurement for the operating state may be performed by the non-invasive measurement device 40 and may also be performed by the external device 30 or the terminal 100 that receives information on the change in the field (e.g., change in intensity of the field) from the non-invasive measurement device 40. In addition to the CCD camera, various methods, such as a blood pressure measurement method, a finger measurement method, a flash method, and a fingerprint (palm) measurement method, may be applied as the external non-invasive measurement device 40.

Meanwhile, the external non-invasive measurement device 40 may perform calibration or may measure the operating state of the implant device 20 according to measurement results and, in addition thereto, may determine an abnormality status according to the operating state of the implant device 20. If a situation requiring removal of the implant device 20 is detected, the external non-invasive measurement device 40 may provide an alarm to the user through the biometric information measurement device 10 and/or the terminal 100.

### (2) Solving problems in structural implementation of an antenna and a sensor

In the case of the conventional implantable biometric information measurement method, various issues arose in terms of sensing capability and data transmission due to structural limitations found in the antenna and the sensor.

The biometric information measurement device 10 according to example embodiments of the present invention may solve the aforementioned problems through the following example embodiments.

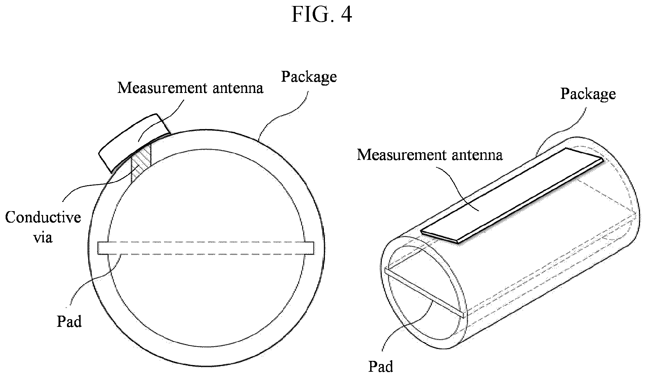

FIG. 4 illustrates a front view and a perspective view of a package of an implant device according to an example embodiment of the present invention. As illustrated in FIG. 4, the implant device 20 according to the example embodiment may be formed as a cylindrical package and a measurement antenna may be provided on the outer surface of the cylindrical package.

Meanwhile, as illustrated in FIG. 4, a pad on which a system on chip (SCO) is formed is inserted into the package. A conductive via may be formed to connect the measurement antenna on the outer surface of the package and the pad on which the SOC is formed inside the package. In detail, the measurement antenna and the pad on which the SOC is formed may be connected by forming the via that penetrates the package and by filling the via with a conductive material. Also, a conducting wire connected at the via may be connected to the pad using a clip-type member. Here, a clip member in a C-clip tension structure may be used as the clip-type member.

Also, as shown in FIG. 4, since an insertion groove is formed on the inner surface of the package to allow the pad to be inserted, the pad may be easily inserted into the package or may be easily removed from the package.

Through this structure, an area of the biometric information measurement device 10 may be minimized, while improving sensing performance and preventing disconnection between the measurement antenna and the SOC.

### (3) Solving a biosafety problem

Since the implant device 20 is a sensor inserted into the body for its use, it is very important to solve the biosafety problem. However, in the case of the conventional implantable sensor, issues regarding biosafety continue to arise.

The biometric information measurement device 10 according to example embodiments of the present invention may solve the aforementioned problem through the following example embodiment.

The outside of the implant device 20 according to an example embodiment may be formed to be wrapped with a material capable of minimizing impact on the permittivity while ensuring biosafety. For example, an external case may be formed with a material, such as silicone, which is proven safe even when inserted into the body, and the package may be accommodated inside the external case. Here, the external case may be formed in a shape in which the package and the measurement antenna formed outside the package may be stably accommodated.

Also, drug or substance capable of suppressing inflammation from occurring after the implant device 20 is inserted into the body may be included on the surface of the external case. For example, when a silicone breast implant is inserted into the body, platelets in the blood are activated and a substance called transforming growth factor (TGF)-β is secreted, which may cause inflammation or fibrosis. Here, when drug, tranilast, is administered to the breast implant, the drug may inhibit TGF-0 and may directly reduce the number of monocytes. Also, when tranilast is loaded into an implant made of polylactic acid-glycolic acid (PLGA) polymer component, the inflammation may be effectively suppressed since the drug may be released over a long period of time.

As describe above, the external case may be applied or coated with an anti-inflammatory substance or drug, and substance or drug, such as pirfenidone, mitomycin, acetylsalicylic acid, tranilast, etc., may be applied. In addition, any substance or drug that has proven anti-inflammatory performance and biosafety may be applied to the external case of the implant device 20.

Also, although the external case is described as an example, it is provided as an example only and, without being limited thereto, if it is a method of wrapping the outside of the implant device 20 with a biosafety material, various methods and structures may be applied.

The biometric information measurement device 10 according to the example embodiment may solve the biosafety problem of the implant device 20 by applying an external case made of a material with the ensured biosafety and a drug or a substance with an anti-inflammatory component.

### (4) Solving a problem in arrangement structure of a coil and an antenna

Since the implant device 20 according to an example embodiment of the present invention uses a permittivity measurement method, there is a need for a structure capable of reducing interference between a radio frequency (RF) antenna for measurement and a power receiving (Rx) coil.

To this end, two structures are proposed.

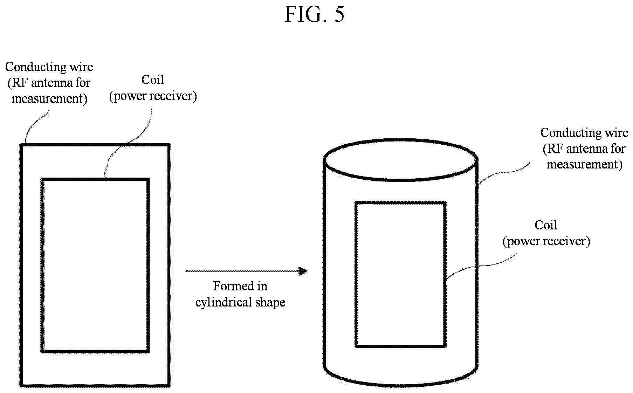

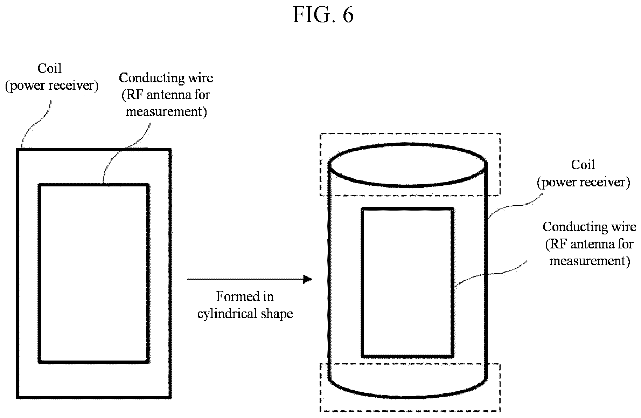

FIGS. 5 and 6 illustrate examples of an arrangement structure of an RF antenna for measurement and a power receiving coil of an implant device.

FIG. 5 illustrates an example in which a conducting wire for an RF antenna for measurement is provided in an outermost area of the implant device 20 and a power receiver including a power Rx coil is provided in an inner center area. On the contrary, FIG. 6 illustrates an example in which the power Rx coil is provided in the outermost area of the implant device 20 and the conducting wire for the RF antenna for measurement is provided in the inner center area. Here, due to influence of an inductance value, more coils may be wound around areas at both ends of the implant device 20.

As described above, the biometric information measurement device 10 according to the example embodiment may reduce interference between the RF antenna for measurement and the power Rx coil through structural division between the RF antenna for measurement and the power Rx coil within the implant device 20.

### (5) Solving a reliability problem regarding measurement accuracy of a sensor

The biometric information measurement device 10 according to example embodiments of the present invention may be applied to the following technology to improve measurement accuracy of the implant device 20.

An additional sensor may be configured for precise measurement of the analyte concentration in the body, but a size of the implant device 20 may be limited in that the implant device 20 is a sensor configured to insert into the body.

Therefore, in the implant device 20 according to the example embodiment, a sensing function may be added to the power receiver such that the power receiver may be responsible for power reception and switched to be in charge of the sensing function, thereby improving sensing accuracy.

In detail, the implant device 20 may be configured to add a sensor function to the power receiver (e.g., wireless power transfer (WPT)) and to temporarily stop power reception and perform sensing as long as there is no problem in receiving power while being responsible for a power receiving function.

In another example, the implant device 20 may be configured with a module that may operate in a total of three modes, a function of a power receiver, a function of a communication unit (e.g., NFC), and a function of a sensor, and may be configured to be operable in three modes with a single module.

Also, the implant device 20 may improve measurement accuracy by using a RF (high frequency data) for analyte concentration measurement and a signal supply RF (low frequency data) together.

Due to the aforementioned configuration, the biometric information measurement device 10 according to example embodiments of the present invention may improve the measurement accuracy without exceeding the size limit of the implant device 20.

### (6) Solving an alignment problem with an external device

The implant device 20 inserts into the body of the subject to be analyzed, and the external device 30 is provided on the skin outside the body of the subject to be analyzed. Therefore, if positions of two sensors are not accurately aligned, data communication may not be smoothly performed.

Therefore, the biometric information measurement device 10 according to example embodiments of the present invention may accurately align the implant device 20 and the external device 30 such that smooth data communication may be performed.

In an example embodiment, to align the implant device 20 and the external device 30, the external device 30 may include a dial capable of adjusting a position of the communication unit 31 for receiving measurement data of the implant device 20.

For example, the biometric information measurement device 10 may provide information on a degree of alignment between the implant device 20 and the external device 30 and may request the external device 30 for adjustment of the dial.

As a detailed example, a first position detection sensor may be included in the implant device 20 and a second position detection sensor may be included in the external device 30. Also, a position adjustment tool capable of finely adjusting a position of the communication unit 31 in the external device 30 may be provided to the external device 30. That is, position state information may include information on a degree of alignment between the external device 30 and the implant device 20 and information on the degree of alignment may be generated based on position detection results of the first position detection sensor included in the implant device 20 and position detection results of the second position detection sensor included in the external device 30. For example, information on the degree of alignment may be generated by the external device 30 or the terminal 100.

Here, the processor 32 of the external device 30 may generate the position state information based on the position detection results of the first position detection sensor and the second position detection sensor and may control the position adjustment tool to compensate for a position error between the communication unit 22 of the implant device 20 and the communication unit 31 of the external device 30.

Depending on example embodiments, the processor 32 of the external device 30 may generate the position state information based on the position detection results of the first position detection sensor and the second position detection sensor. Here, if a position state value exceeds a threshold, the processor 32 may determine that a position of the external device 30 is too far from the implant device 20 and may request the user to relocate the external device 30. For example, if a position state value between a first position of the external device 30 and a second position of the implant device 20 according to the position detection results of the first position detection sensor included in the implant device 20 and the position detection results of the second position detection sensor included in the external device 30 is greater than or equal to the threshold, the external device 30 may generate and output an alarm message for requesting the user to relocate the external device 30 or may deliver the same to the terminal 100.

Meanwhile, although an example in which the position adjustment tool of the communication unit 31 is provided in the external device 30 is described, it is provided as an example only and, without being limited thereto, a position adjustment tool of the communication unit 22 may be provided to the implant device 20 depending on example embodiments.

Also, the aforementioned position adjustment tool is only an example of various methods of aligning a position between the implant device 20 and the external device 30 and the present invention is not limited thereto. Various methods may be applied as long as they do not affect measurement performance of the sensor.

Hereinafter, additional example embodiments in addition to the aforementioned example embodiments will be described.

The implant device 20 of the biometric information measurement device 10 according to an example embodiment may include a plurality of measurement sensors to improve accuracy and may additionally include various sensors capable of measuring temperature and humidity. Here, the biometric information measurement device 10 may further include a correction module configured to correct measurement results using measurement data from the plurality of measurement sensors. The correction module may be configured with a measurement result correction algorithm to improve accuracy and may correct a value of analyte concentration using data measured from the plurality of measurement sensors or measurement data for different metrics.

In another example embodiment, the biometric information measurement device 10 requires a method for reducing foreign body sensation or pain until the implant device 20 is stabilized in the body of the subject to be analyzed when the implant device 20 is inserted into the body of the subject to be analyzed. Therefore, the implant device 20 may be coated with a substance that induces stabilization in the body or the substance may be inserted into the implant device 20 in a capsule form. In this way, the implant device 20 in a state in which the material that induces stabilization inside the body is coated or inserted in the capsule form may reduce foreign body sensation and pain of the subject while the material inserted into the body of the subject to be analyzed is being decomposed and may resolve inconvenience of the subject to be analyzed until the implant device 20 is stabilized.

In still another example embodiment, the biometric information measurement device 10 may calculate analyte concentration results based on correlation between a change in a resonant frequency and a change in analyte concentration using an analyte concentration calculation algorithm. The biometric information measurement device 10 may calculate the analyte concentration using a resonant frequency corresponding to a change in the subcutaneous permittivity. Here, for various reasons, undesired surrounding tissue may encompass the implant device 20, which may cause the change in the permittivity due to an increase in a surrounding tissue layer. Here, the biometric information measurement device 10 may use the analyte concentration calculation algorithm to exclude influence of other factors on a resonant frequency value. However, without being limited to the analyte concentration calculation algorithm, sensor measurement technology capable of measuring analyte concentration while excluding the influence of other factors may be applied.

In still another example embodiment, the analyte concentration measured by the biometric information measurement device 10 is affected by food consumed by the subject to be analyzed. For example, blood sugar is largely affected by food consumed by the subject to be analyzed. Therefore, the subject to be analyzed may be provided with a service that calculates an amount of food consumed by the subject to be analyzed and predicts and manages a change in the analyte concentration of the subject to be analyzed based on the calculated amount of food. This service may be provided by the terminal 100 or the server 200 that collects and manages the analyte concentration for each subject to be analyzed. For example, the service may be provided by a service application installed and running on the terminal 100 or through communication with the server 200.

As a more detailed example, the server 200 may receive a pre-meal image and a post-meal image captured by the terminal 100. Here, the pre-meal image may represent an image of food taken with the terminal 100 before the subject to be analyzed eats and the post-meal image may represent an image of food taken with the terminal 100 after the subject to be analyzed finishes eating. The server 200 may analyze the pre-meal image to calculate an amount and volume of at least one food included in the pre-meal image and calculate calories of each food included in the pre-meal image and total calories of food. Afterwards, when the post-meal image is received, the server 200 may calculate an amount and volume of at least one food in the post-meal image and calculate calories of each food included in the post-meal image and total calories of food. Here, the server 200 may calculate an intake amount, total calories, and sugar consumed by the subject to be analyzed by comparing analysis results of the pre-meal image and analysis results of the post-meal image and, based thereon, may predict a change in analyte concentration of the subject to be analyzed.

In an example embodiment, the server 200 may calculate measurement accuracy by comparing a change prediction value of analyte concentration and a value of analyte concentration measured by the biometric information measurement device 10. To secure the measurement accuracy, it is desirable to assume that any other food other than the above meal is not consumed by the subject to be analyzed.

In another example embodiment, when the pre-meal image is received from the terminal 100 of the subject to be analyzed, the server 200 may calculate an amount of food the subject needs to consume using the current analyte concentration of the subject to be analyzed or change trend of today's analyte concentration measured by the biometric information measurement device 10 and may provide meal amount suggestion information. For example, with information on today's analyte concentration of the subject to be analyzed or change trend of today's analyte concentration, the server 200 may suggest to the subject to be analyzed with an amount of meal to be eaten in the food included in the pre-meal image. For example, if the subject to be analyzed's analyte concentration or change trend of the analyte concentration for the corresponding day is not good, the server 200 may suggest that the subject to be analyzed eat a certain amount rather than all of the meal shown in the pre-meal image, thereby preventing the subject to be analyzed from overeating.

Also, the server 200 may also suggest exercise, an activity level, and a lifestyle habit, in addition to a meal amount and an eating habit of the subject to be analyzed, using intake amount information of the subject to be analyzed calculated as above. For example, if an intake amount for a predetermined period of time (e.g., 1 day, 1 week, etc.) is larger than the average meal amount of the subject to be analyzed, the server 200 may suggest to the subject with exercise, activity, lifestyle habit, etc. that may increase a basal metabolic rate.

Also, the server 200 may receive biometric measurement data (e.g., heart rate, oxygen saturation data, etc.) of the subject from a wearable device (e.g., smart watch, etc.) used by the subject to be analyzed, may analyze the same, and may use the analyzed data to manage the analyte concentration. For example, the server 200 may calculate the average basal metabolic rate of the subject to be analyzed using data received from the wearable device of the subject to be analyzed, may also analyze the average meal amount of the subject to be analyzed, may analyze problems with high analyte concentration of the subject to be analyzed, and may provide the analyzed problems and tips to improve the problems.

In an example embodiment, if the measured analyte concentration of the subject to be analyzed rapidly increases to be greater than or equal to a threshold or if the change trend of analyte concentration rapidly changes by the threshold or more, the server 200 and/or the biometric information measurement device 10 may provide an alarm to the external device 30 or the terminal 100. Here, the alarm may be provided in a manner that the subject to be analyzed may quickly identify, such as vibration, sound, and light.

In addition, the server 200 may receive a selection on a guardian terminal of the subject to be analyzed and, in a situation of providing the alarm, may provide the alarm to the guardian terminal to check the subject to be analyzed.

In addition, any method capable of providing an alert about the analyte concentration to the subject to be analyzed or the guardian of the subject to be analyzed may be applied.

In an example embodiment, the biometric information measurement device 10 may include sensors, such as a motion sensor, a vibration sensor, and a gyro sensor, to generate movement data of the subject to be analyzed and detect movement of the subject to be analyzed. If there is no change in a movement of the subject to be analyzed for a preset period of time, the biometric information measurement device 10 may switch a sensor of the implant device 20 and/or the external device 30 to a power saving mode (sleep mode).

Also, the server 200 may determine a rest state and a sleep state of the subject using data measured and received from the wearable device worn by the subject to be analyzed.

Also, the server 200 may receive movement data of the subject to be analyzed measured by the biometric information measurement device 10 and biometric data measured from the wearable device, may determine the same, and when the movement data and the biometric data of the subject to be analyzed are determined as an unstable state, may recognize a corresponding situation as an emergency situation and may generate an emergency alarm.

Also, the biometric information measurement device 10 may further provide a temperature sensor capable of measuring body temperature of the subject to be analyzed to the implant device 20 and/or the external device 30 and may use a measured temperature value.

In an example embodiment, in the case of inserting the implant device 20 into a companion animal as a subject to be measured, a method capable of fixing a position of the implant device 20 without moving may be required. To this end, a fixing tool may be added to the implant device 20 such that the implant device 20 may be fixed at an initially inserted position.

In addition, a position detection sensor capable of detecting an alignment status with the implant device 20 may be further configured in the external device 30. Here, if a position of the implant device 20 is moved by a predetermined distance, position adjustment of the external device 30 may be requested by providing information on the moved distance.

As another example, although the implant device 20 is tilted by a certain tilt or more relative to the skin surface or a position of the implant device 20 is moved by a threshold or more, an alarm message may be provided to the external device 30 and/or the terminal 100 to notify the user to perform corresponding correction or to undergo inspection. For example, the implant device 20 may include a tool configured to measure a tilt of the implant device 20 based on the skin surface and a tool configured to provide an alarm through the external device 30 or the terminal 100 when the measured tilt is tilted by a predetermined tilt or more.

Since the implant device 20 is to insert into the body of the subject to be analyzed, a specific insertion device or insertion method may also be added. As described above, a structure and a method capable of stably inserting a sensor (e.g., implant device 20) into the body of the subject to be analyzed may be applied to a device and a method for inserting a sensor for measuring biometric information, such as analyte concentration, into the body. In addition, a substance to improve stability in the body during insertion may be further included.

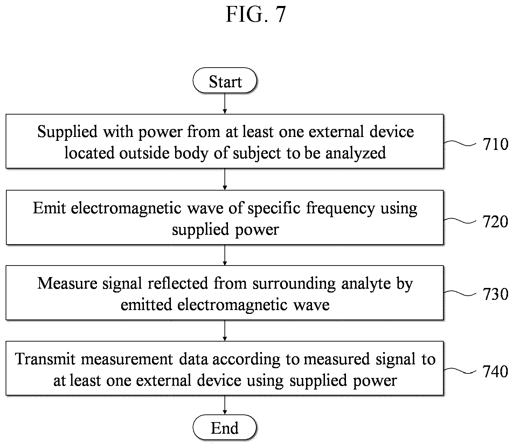

FIG. 7 is a flowchart illustrating an example of a biometric information measurement method of an implant device according to an example embodiment of the present invention.

In operation 710, the implant device 20 may be supplied with power from at least one external device located outside a body of a subject to be analyzed. Here, each of the at least one external device may correspond to the external device 30. Here, the implant device 20 may be supplied with the power from the at least one external device through wireless power transfer (WPT).

In operation 720, the implant device 20 may emit an electromagnetic wave of a specific frequency using the supplied power. In an example embodiment, the implant device 20 may include a package, a conductive via formed in at least a portion of the package to connect inside and outside of the package, a measurement antenna connected to the conductive via on the outside of the package, a pad formed in the package and in which an SOC is formed, and a conducting wire configured to connect the via and the pad. In this case, the implant device 20 may deliver at least a portion of the supplied power to the measurement antenna through the conducting wire and the conductive via such that the measurement antenna may emit the electromagnetic wave of the specific frequency. In another example embodiment, the implant device 20 may include a measurement antenna conducting wire provided along an outermost area of the package of the implant device 20 and a power Rx coil provided in a center area of the package, spaced apart from the measurement antenna conducting wire, or may include a power Rx coil provided along an outermost area of the package of the implant device 20 and a measurement antenna conducting wire provided in a center area of the package, spaced apart from the power Rx coil. In this case, the implant device 20 may deliver at least a portion of the supplied power to the measurement antenna conducting wire such that the measurement antenna may emit the electromagnetic wave of the specific frequency. In still another example embodiment, the implant device 20 may include a coil having a sensing function and a power receiving function, and may be configured to switch between the sensing function and the power receiving function and to switch power reception from the at least one external device and the sensing function according to measurement of the reflected signal with emission of the electromagnetic wave. In this case, the implant device 20 may be supplied with the power through the power receiving function in operation 710 and may switch the power receiving function to the sensing function and then emit the electromagnetic wave of the specific frequency using the power supplied through the power receiving function in operation 720.

In operation 730, the implant device 20 may measure a signal reflected from surrounding analyte by the emitted electromagnetic wave. As described above, if the analyte concentration (e.g., blood sugar level) in the blood vessel of the subject to be analyzed changes, the analyte concentration in a subcutaneous area may also change. In this case, permittivity in the subcutaneous area may vary according to the change in the analyte concentration. Here, a resonant frequency emitted by the implant device 20 may vary depending on a change in the permittivity in the surrounding subcutaneous area. For example, the measurement unit 21 of the implant device 20 may include a specific pattern of a conducting wire and a feeder. Here, if the permittivity in the surrounding subcutaneous area varies, capacitance of the measurement unit 21 may also vary and thus, the resonant frequency may also vary due to the specific pattern and the feeder. Since the subcutaneous analyte concentration changes in proportion to the analyte concentration of adjacent blood vessel, the biometric information measurement device 10 may measure a signal reflected from the surrounding analyte through a resonant frequency corresponding to the change in the subcutaneous permittivity.

In operation 740, the implant device 20 may transmit measurement data according to the measured signal to the at least one external device using the supplied power. For example, the implant device 20 may transmit the measurement data according to the measured signal to the at least one external device through the communication unit 22.

Meanwhile, the at least one external device may include a first external device and a second external device arranged at a predetermined interval outside the body of the subject to be analyzed, and the first external device and the second external device may be coupled to measure an electromagnetic wave according to a change in the analyte concentration in the interstitial fluid on the outer surface of the skin of the subject to be analyzed. In this case, correction may be performed on a measurement value of analyte concentration using measurement data measured by the implant device and the electromagnetic wave measured by the first external device and the second external device. The correction may be performed by the first external device, the second external device, the terminal 100, or the server 200.

Also, the at least one external device may perform measurement on the analyte by emitting an electromagnetic wave that reaches a depth of the blood vessel of the subject to be analyzed and by measuring a signal that reaches the blood vessel of the subject to be analyzed and is reflected from the analyte. It corresponds to the aforementioned mode 3 and may solve a problem coming from a time delay.

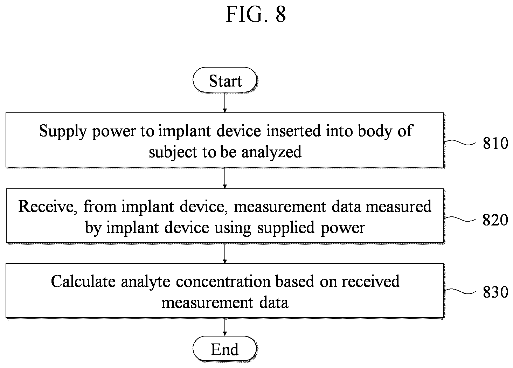

FIG. 8 is a flowchart illustrating an example of a biometric information measurement method of another external device according to an example embodiment of the present invention.

In operation 810, the external device 30 may supply power to the implant device inserted into a body of a subject to be analyzed. Here, the external device 30 may supply power to the implant device 20 through WPT.

In operation 820, the external device 30 may receive, from the implant device 20, measurement data measured by the implant device 20 using the supplied power. As described above, if analyte concentration (e.g., blood sugar value) in the blood vessel of the subject to be analyzed changes, analyte concentration in a subcutaneous area may change. In this case, the permittivity in the subcutaneous area may vary according to the change in the analyte concentration. Here, a resonant frequency emitted from the implant device 20 may vary according to the change in the permittivity in the surrounding subcutaneous area. For example, the measurement unit 21 of the implant device 20 may include a specific pattern of a conducting wire and a feeder. Here, if the permittivity in the surrounding subcutaneous area varies, capacitance of the measurement unit 21 may also vary and thus, the resonant frequency may also vary due to the specific pattern and the feeder. Since the subcutaneous analyte concentration changes in proportion to the analyte concentration of adjacent blood vessel, the biometric information measurement device 10 may measure a signal reflected from surrounding analyte through a resonant frequency corresponding to the change in the subcutaneous permittivity.

In operation 830, the external device 30 may calculate the analyte concentration based on the received measurement data. A method of calculating the analyte concentration is described above in detail. Also, depending on example embodiments, the external device 30 may transmit the received measurement data to the terminal 100 such that the analyte concentration may be calculated by the terminal 100.

The external device 30 may be coupled to another external device provided at a predetermined interval outside the body of the subject to be analyzed to measure an electromagnetic wave according to a change in concentration of an analyte in the interstitial fluid on the outer skin of the subject to be analyzed. In this case, correction may be performed on a measurement value of analyte concentration using measurement data measured by the implant device 20 and the electromagnetic wave measured by the external device 30 and the other external device. As described above, the correction may be performed by the first external device, the second external device, the terminal 100, or the server 200.

Also, the external device 30 may perform measurement on the analyte by emitting an electromagnetic wave that reaches a depth of the blood vessel of the subject to be analyzed and by measuring the signal that reaches the blood vessel of the subject to be analyzed and is reflected from the analyte. In this case, the electromagnetic wave that reaches the depth of the blood vessel of the subject to be analyzed may be formed through coupling between two or more external devices.

As described above, according to example embodiments of the present invention, there may be provided a device and method for measuring biometric information that may variously use an implant device configured to insert into a body of a subject to be analyzed and to measure biometric information.

The systems or the apparatuses described herein may be implemented using hardware components or combination of hardware components and software components. For example, the apparatuses and the components described herein may be implemented using one or more general-purpose or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of responding to and executing instructions in a defined manner. A processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that the processing device may include multiple processing elements and/or multiple types of processing elements. For example, the processing device may include multiple processors or a processor and a controller. In addition, different processing configurations are possible, such as parallel processors.

The software may include a computer program, a piece of code, an instruction, or at least one combination thereof, for independently or collectively instructing or configuring the processing device to operate as desired. Software and/or data may be embodied in any type of machine, component, physical equipment, virtual equipment, or computer storage medium or device, to provide instructions or data to or to be interpreted by the processing device. The software also may be distributed over network coupled computer systems so that the software is stored and executed in a distributed fashion. The software and data may be stored by one or more computer readable storage mediums.

The methods according to the above-described example embodiments may be configured in a form of program instructions performed through various computer devices and recorded in computer-readable media. The media may also include, alone or in combination with the program instructions, data files, data structures, and the like. The media may continuously store a computer-executable program or may temporarily store the program for execution or download. Also, the media may be various recording methods or storage methods in which one or a plurality of hardware is combined and may be present on a network in a distributed manner without being limited to media directly connected to a computer system. Examples of the media include magnetic media such as hard disks, floppy disks, and magnetic tapes; optical media such as CD ROM disks and DVDs; magnetooptical media such as floptical disks; and hardware devices that are to store program instructions, such as read-only memory (ROM), random access memory (RAM), flash memory, and the like. Also, examples of other media may include recording media and storage media managed by an app store that distributes an application or a site, a server, etc., that supplies and distributes other various software. Examples of program instructions include both machine code, such as produced by a compiler, and files containing higher level code that may be executed by the computer using an interpreter. MODE

While the example embodiments are described with reference to specific example embodiments and drawings, it will be apparent to one of ordinary skill in the art that various changes and modifications in form and details may be made in these example embodiments from the description. For example, suitable results may be achieved if the described techniques are performed in a different order, and/or if components in a described system, architecture, device, or circuit are combined in a different manner, or replaced or supplemented by other components or their equivalents.

Therefore, other implementations, other example embodiments, and equivalents of the claims are to be construed as being included in the claims.

## Claims

1. A sensor information measurement device comprising:
an implant device configured to insert into a body of a subject to be analyzed, to emit an electromagnetic wave of a specific frequency, and to measure a signal reflected from a surrounding analyte; and
a non-invasive measurement device configured to measure a change in a field formed by the electromagnetic wave of the specific frequency outside the body of the subject to be analyzed.

2. The sensor information measurement device of claim 1, wherein the non-invasive measurement device is configured to measure a change in intensity of the field that changes according to a change in permittivity in the body of the subject to be analyzed.

3. The sensor information measurement device of claim 1, wherein the non-invasive measurement device is configured to process at least one of correction on measurement data of the implant device and measurement of an operating state of the implant device based on the measured change in the field.

4. The sensor information measurement device of claim 3, wherein the non-invasive measurement device is configured to determine an abnormality status of the implant device according to the operating state of the implant device and to provide an alarm to a user through the sensor information measurement device or a terminal that communicates with the sensor information measurement device depending on the determined abnormality status.

5. The sensor information measurement device of claim 1, further comprising:
an external device configured to supply power to the implant device, to receive first measurement data of the implant device, to receive the change in the field measured by the non-invasive measurement device, and to process at least one of correction on measurement data of the implant device and measurement of an operating state of the implant device based on the received change in the field.

6. The sensor information measurement device of claim 5, wherein the external device includes:
a communication unit configured to communicate with the implant device; and
a position adjustment tool configured to adjust a position of the communication unit in the external device based on position state information with the implant device.

7. The sensor information measurement device of claim 6, wherein the position state information includes information on a degree of alignment between the external device and the implant device, and
information on the degree of alignment is generated based on position detection results of a first position detection sensor included in the implant device and position detection results of a second position detection sensor included in the external device.

8. The sensor information measurement device of claim 7, wherein the external device is configured to generate an alarm message for requesting a user to relocate the external device if a position state value between a first position of the external device and a second position of the implant device according to the position detection results of the first position detection sensor and the position detection results of the second position detection sensor included in the external device is greater than or equal to a threshold.

9. The sensor information measurement device of claim 1, wherein the implant device includes a fixing tool configured to fix the implant device at an insertion position in the body of the subject to be analyzed.

10. The sensor information measurement device of claim 1, wherein the implant device includes:
a tool configured to measure a tilt of the implant device based on the skin surface of the subject to be analyzed; and
a tool configured to provide an alarm through an external device or a terminal when the measured tilt is tilted by a predetermined tilt or more.
